# EUROPÄISCHE PATENTSCHRIFT

(11) **EP 2 907 463 B1**
(45) Veröffentlichungstag und Bekanntmachung des Hinweises auf die Patenterteilung: **20.04.2016**
(21) Anmeldenummer: 14154832.1
(22) Anmeldetag: 12.02.2014
(51) Int. Cl.: A61B 18/14, A61B 18/00

(54) **Chirurgisches Instrument mit Elektrodenträger**
Surgical instrument with electrode holder
Instrument chirurgical doté d'un support d'électrodes

(43) Veröffentlichungstag der Anmeldung: 19.08.2015
(73) Patentinhaber: ERBE Elektromedizin GmbH, 72072 Tübingen (DE)
(72) Erfinder: Mayer, Dr. Volker, 72072 Tübingen (DE); Schmid, Markus, 73092 Heiningen (DE); Brodbeck, Achim, 72555 Metzingen (DE)
(74) Vertreter: Rüger, Barthelt & Abel

(56) Entgegenhaltungen:
- EP-A1- 2 377 480
- EP-A1- 2 554 132
- EP-A1- 2 674 124
- DE-A1-102011 003 520

## Beschreibung

Die Erfindung betrifft ein chirurgisches Instrument, zur elektrochirurgischen mono- oder bipolaren Applikation von Strom auf biologisches Gewebe, insbesondere ein Versiegelungsinstrument.

Instrumente zur Koagulation von biologischem Gewebe zwischen zwei Branchen eines Werkzeugs mit wenigstens einer beweglichen Branche sind aus dem Stand der Technik bekannt. Die EP 2 554 132 zeigt dazu ein Instrument, dessen Branchen jeweils einen Elektrodenträger und eine dünne plattenförmige Elektrode aufweist. Der Elektrodenträger kann aus einem massiven Metallteil oder einem kunststoffummantelten Metallteil bestehen. Die Elektrode ist mit dem Elektrodenträger über mehrere punktartige Schweißverbindungen verbunden. Damit soll einerseits eine sichere mechanische Verbindung und andererseits ein lediglich geringer Wärmeübertrag zwischen der Elektrode und dem Elektrodenträger erzielt werden. Die Schweißnähte haben zwar nur einen geringen Durchmesser, aber auch eine lediglich geringe Länge (Ausdehnung in Wärmeflussrichtung), was ihre Wirkung als Wärmebarriere begrenzt.

Einerseits muss eine mechanisch stabile und elektrisch sichere Verbindung zwischen der Elektrodenplatte und dem Elektrodenträger geschaffen werden. Andererseits soll der Wärmeübertrag minimiert werden.

Davon ausgehend ist es Ziel der Erfindung, ein verbessertes chirurgisches Instrument insbesondere mit einer verbesserten Branche für den offenchirurgischen, laparoskopischen und endoskopischen Einsatz anzugeben.

Diese Aufgabe wird mit dem chirurgischen Instrument, insbesondere einem elektrochirurgischen Instrument nach Anspruch 1 gelöst:

Das erfindungsgemäße Instrument weist eine Branche auf, bei der der Elektrodenträger und die Elektrodenplatte über mehrere Stege nahtlos einstückig miteinander verbunden sind. Somit sind die Stege und auch die Übergänge zwischen den Stegen und dem Elektrodenträger einerseits, und der Elektrodenplatte andererseits, übergangslos aus Material gleicher Zusammensetzung und Struktur ausgebildet. Dabei weist das bevorzugte Material hinreichend elektrisch leitfähige Eigenschaften auf, sodass mittels der Elektrodenplatte Strom an Gewebe appliziert werden kann. Schweißnähte, wie sie durch Wiederaufschmelzung von Teilen einer Elektrodenplatte oder eines Branchenträgers bei Fertigung aus mehreren Einzelteilen entstehen, fehlen hier völlig. Dieses Konzept ermöglicht die Minimierung der Dimensionen der Stege auf das elektrisch, mechanisch und fertigungstechnisch Notwendige. Es ermöglicht außerdem eine Maximierung der Länge der Stege. Vorzugsweise ist die Länge eines Steges wenigstens so groß wie die Quadratwurzel aus seiner durchschnittlichen Querschnittsfläche. Weiter vorzugsweise ist die Länge eines Steges wenigstens so groß wie die Quadratwurzel aus seiner geringsten Querschnittsfläche. Durch diese Maßnahmen kann der Wärmeübergangswiderstand von der Elektrodenplatte zu dem Elektrodenträger maximiert werden. Dies insbesondere, wenn ein in den Schlitz zwischen dem Elektrodenträger und der Elektrodenplatte eingespritzter Kunststoff die mechanische Verbindungswirkung der Stege unterstützt oder auch weitgehend übernimmt.

Die Ausbildung eines möglichst großen Wärmewiderstands zwischen der Elektrodenplatte und dem Elektrodenträger kann dazu genutzt werden, die von der Elektrodenplatte abgewandte Seite der Branche in Betrieb möglichst kühl zu halten. Während sich das zwischen Elektroden gefasste Gewebe und somit auch die damit in Kontakt stehende Elektrodenplatte auf Temperaturen von über 100°C erhitzen kann, kann der Elektrodenträger und damit die rückseitige Außenseite der Branche auf niedriger, eine Schädigung am Gewebe verhindernder oder zumindest reduzierender, Temperatur im Vergleich zu der Elektrodenplatte gehalten werden. Eine Gewebeschädigung kann beispielsweise bereits ab 40°C mindestens doch ab 60°C eintreten. Dies ermöglicht eine punktgenaue und gezielte Gewebebehandlung auch bei schwierigen Eingriffen und in unmittelbarer Nachbarschaft zu empfindlichem Gewebe, wie beispielsweise Nervengewebe.

Bei einer speziellen Ausführungsform sind die Stege von der Mündung des Schlitzes beabstandet angeordnet. Dadurch wird der geringe Wärmefluss von der Elektrodenplatte zu dem Elektrodenträger vom Rand des Elektrodenträgers ferngehalten, so dass die Randtemperatur der Branche weiter gesenkt werden kann.

Außerdem kann der Abstand der Stege von der Randkontur des Elektrodenträgers größer als die Weite des Schlitzes sein. Dies fördert den oben genannten Effekt.

Die Stege können einen Rundquerschnitt aufweisen. Es ist aber auch möglich, dass diese einen abweichenden unrunden Querschnitt aufweisen, wobei die Querschnitte aller Stege gleich oder auch unterschiedlich ausgebildet sein können. Die Querschnitte der Stege können außerdem gleiche oder unterschiedliche Orientierungen aufweisen, um bspw. die Querstabilität der Lagerung der Elektrodenplatte an dem Elektrodenträger zu maximieren.

Die Stege können einen größten Durchmesser aufweisen, der kleiner ist als die Schlitzweite. Die Stege sind dann sehr filigran und haben eine geringe Wärmeleitfähigkeit. Wenn der Elektrodenträger einen schalenförmigen Querschnitt aufweist, kann die Länge der einzelnen Stege maximiert werden, was den Wärmewiderstand weiter erhöht.

Außerdem kann die Elektrodenplatte an einem Ende einstückig, nahtlos in den Elektrodenträger übergehen. Die Elektrodenplatte kann ein ebenes gegebenenfalls profiliertes Teil sein. Die Gestaltung der dem Gewebe zugewandten Elektrodenfläche kann, je nach Anwendungsfall, frei gestaltet werden. Insbesondere kann die Elektrodenplatte einen umlaufenden Rand aufweisen, der die Schlitzweite an der Mündung des Schlitzes verringert.

Vorzugsweise sind der Elektrodenträger, die Stege und die Elektrodenplatte in einem additiven bzw. generativen Fertigungsverfahren hergestellt. Vorzugsweise bestehen sie dabei aus einem einheitlichen Material. Als additives Fertigungsverfahren eignet sich insbesondere das Selektive Laser Melting (SLM), bei dem sowohl die Elektrodenplatte, die Stege sowie der Elektrodenträger aus Metallpulver durch Lasersintern bzw. Laserschmelzen erzeugt werden. Damit haben die Elektrodenplatte, die Stege und der Elektrodenträger eine einheitliche Feinstruktur. Die erzielbaren Materialfestigkeiten sind material- und verfahrensbedingt hoch und vergleichbar zu Gussverfahren. Als weiteres Fertigungsverfahren kommt bei Strukturen mit wenigen Hinterschnitten auch beispielsweise das Metal-Injection-Molding-Verfahren, MIM-Verfahren, in Betracht. Eine erhöhte Oberflächenrauheit der Stege, des Elektrodenträgers und zumindest der dem Elektrodenträger zugewandten Seite der Elektrodenplatte ermöglichen eine feste Haftung von Kunststoff auf diesen Oberflächen. Insbesondere wenn der zwischen der Elektrodenplatte und dem Elektrodenträger gebildete Schlitz mit Kunststoff ausgespritzt und gegebenenfalls der Elektrodenträger auch ansonsten mit Kunststoff umspritzt bzw. umhüllt ist, wird eine innige Metall-Kunststoff-Verbindung erzielt. Dies ist insbesondere im Hinblick auf die hygienischen Anforderungen an chirurgische Instrumente wie auch im Hinblick auf eventuelle Reinigungs- und Sterilisationszyklen vorteilhaft, bei denen das Instrument, insbesondere die Branche, hohen thermischen und auch chemischen Belastungen ausgesetzt wird.

Die Umspritzung des Elektrodenträgers mit Kunststoff bewirkt zumindest eine elektrische und je nach Kunststoffdicke auch eine spürbare thermische Isolierung, was vorteilhaft ist.

Weiter kann die Kunststoffumspritzung zur Erbringung einer mechanischen Kalibrierung des Elektrodenträgers, beispielsweise im Bereich seiner Lagerbohrung, genutzt werden. Der Elektrodenträger weist dazu einen Querdurchgang auf, dessen Präzision bei der Fertigung von untergeordneter Bedeutung ist. Die präzise Lagerbohrung kann dann beim Umspritzen des Elektrodenträgers mit Kunststoff in der Kunststoffspritzgussform durch Nutzung eines Formenkerns erreicht werden, der sich durch die Queröffnung des Elektrodenträgers erstreckt und die Position der Lagerbohrung im Kunststoff präzise festlegt. Immer wenn sich die vorstehenden und die nachfolgenden Beschreibung, sowie die Ansprüche auf Kunststoff bzw. Kunststoffmaterial beziehen, umfasst dies auch Materialien mit isolierenden Eigenschaften, die nicht der Gruppe der Kunststoffmaterialien zugeordnet werden können.

Weitere Einzelheiten vorteilhafter Weiterbildungen ergeben sich aus der Zeichnung, aus Ansprüchen oder aus der Figurenbeschreibung. Es zeigen:
Figur 1 das erfindungsgemäße Instrument, in perspektivischer Übersichtsdarstellung,
Figur 2 das Werkzeug des Instruments nach Figur 1, in perspektivischer Übersichtsdarstellung,
Figur 3 eine Branche des Werkzeugs nach Figur 2, in Seitenansicht,
Figur 4 die Branche nach Figur 3, in Draufsicht,
Figur 5 und 6 Querschnitte verschiedener Ausführungsformen von Branchen entsprechend Figur 3 und 4,
Figur 7 und 8 Querschnitte von Stegen in verschiedenen Ausführungsformen,
Figur 9 einen Querschnitt eines Hohlstegs,
Figur 10 einen Längsschnitt durch einen Hohlsteg mit einem darin aufgenommenen Abstandshalteelement,
Figur 11 einen Horizontalschnitt durch eine Branche nach Figur 3 oder 4 mit Kunststoffummantelung im Bereich der Lagerbohrung,
Figur 12 einen Querschnitt einer abgewandelten Ausführungsform einer erfindungsgemäßen Branche.

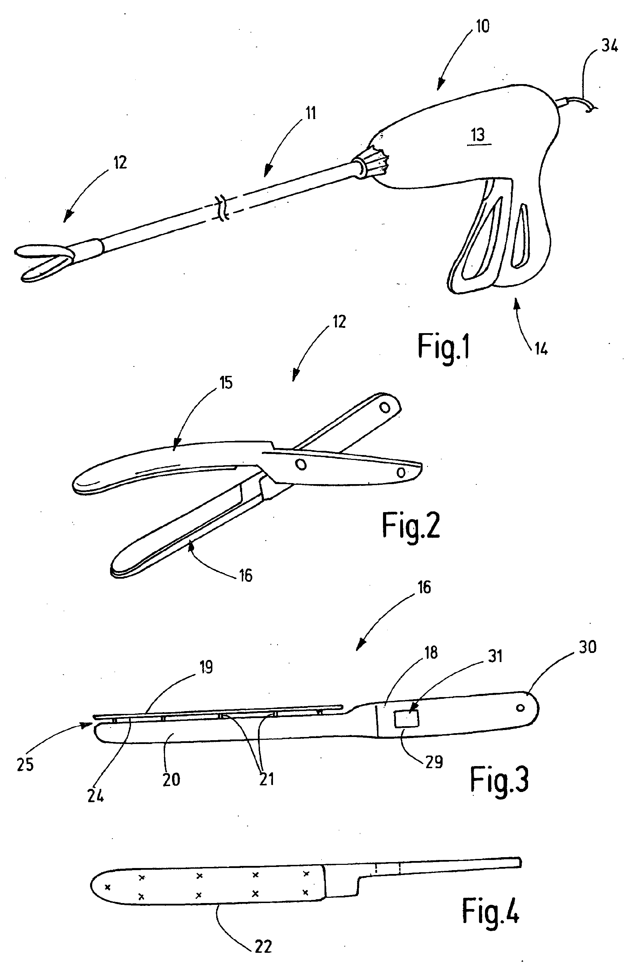

Das in Figur 1 veranschaulichte Instrument 10 ist beispielshalber als Rohrschaftinstrument für den offenchirurgischen und/oder laparoskopischen Einsatz dargestellt. Es weist einen Schaft 11 auf, an dessen distalen Ende ein Werkzeug 12 angeordnet ist. Zur Handhabung des Instruments 10 dient ein Gehäuse 13, das einen Griff 14 aufweist und mit dem proximalen Ende des Schafts 11 verbunden ist. Das Instrument 10 kann jedoch auch als flexibel endoskopisches Instrument ausgebildet sein, wobei dann das Werkzeug 12 sowie der Schaft 11 entsprechend klein und filigran und der Schaft 11 flexibel sind. Die nachfolgende prinzipielle Beschreibung gilt auch für solche Ausführungsformen.

Figur 2 veranschaulicht das Werkzeug 12 mit zwei Branchen 15, 16, die nach Art einer Zange zusammenwirken und prinzipiell einen gleichen Grundaufbau aufweisen. Die nachfolgende Beschreibung der in den Figuren 3 bis 6 dargestellten Branche 16 gilt deswegen entsprechend für die Branche 15.

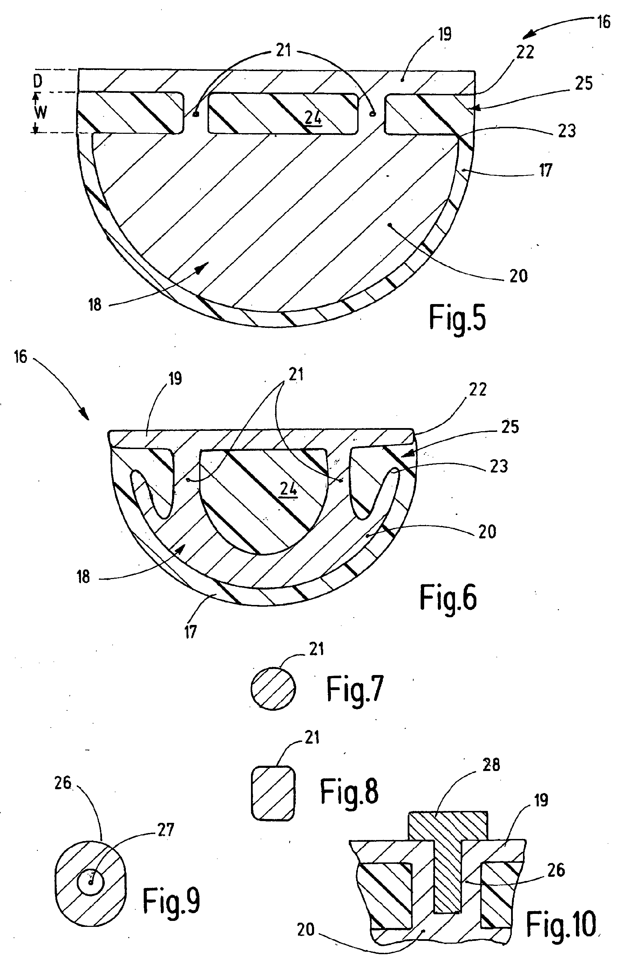

Die Branche 16 ist in Figur 4 unter Weglassung ihres Kunststoffmantels 17 veranschaulicht, der seinerseits in Figur 5 detaillierter dargestellt ist.

Figur 3 und 4 veranschaulichen somit lediglich den aus Metall bestehenden Teil der Branche 16. Dieses Metallteil 18 ist in eine nach Art eines dünnen, ggf. umgeformten Blechs ausgebildete Elektrodenplatte 19, einen Elektrodenträger 20 und mehrere dazwischen angeordnete verbindende Stege 21 gegliedert. Die Elektrodenplatte 19, der Elektrodenträger 20 und die Stege 21 sind nahtlos aus einheitlichem Material einstückig ausgebildet. Die Elektrodenplatte 19 kann, wie dargestellt, eben und nach Figur 4 gerade gestreckt ausgebildet sein. Sie kann jedoch auch in einer oder mehreren Richtungen gekrümmt ausgebildet sein, wenn dies für eine bestimmte Anwendung gewünscht wird. Beispielsweise kann die Elektrode 19 in Draufsicht, abweichend von Figur 4, einem Bogen folgend ausgebildet sein. Außerdem kann sie konvex oder konkav schalenförmig, und falls gewünscht, in jedem der genannten Fälle profiliert ausgebildet sein. Das Profil kann aus Zähnen, Querstegen, Längsrippen längs der Randkontur 22 oder dergleichen bestehen. Weiter ist es möglich, die Elektrodenplatte 19 mit einem durchgehenden oder kurz vor dem distalen Ende endenden Schlitz zur Aufnahme eines Messers zu versehen. Jedenfalls weist die Elektrodenplatte 19 eine sich entlang einer Flanke, dann über das distale Ende und dann entlang der gegenüber liegenden Flanke erstreckenden Randkontur 22 auf (siehe auch Figur 5), die vorzugsweise parallel zu einer Randkontur 23 des Elektrodenträgers 20 verlaufend ausgebildet ist.

Der Elektrodenträger 20 kann, wie Figur 5 zeigt, als Vollprofil ausgebildet sein, und sich gemäß Figur 3 in konstantem oder auch wechselndem Abstand zu der Elektrodenplatte 19 erstrecken. Zwischen einander legen die Elektrodenplatte 19 und der Elektrodenträger 20 einen Schlitz 24 fest, dessen Mündung 25 ringsum entlang der Randkonturen 22 und 23 zwischen diesen festgelegt ist. Wie ersichtlich, kann die Mündung 25 ringsum eine einheitliche Weite W, gemessen zwischen den Randkonturen 22 und 23 aufweisen. Außerdem kann die Randkontur 23, bezogen auf die Randkontur 22, gemäß Figur 5 nach innen hin versetzt sein. Hier überragt die Elektrodenplatte 19 den Elektrodenträger 20. Die Verhältnisse können jedoch auch umgekehrt sein. Die Elektrodenplatte (19) kann eine Dicke (D) aufweisen, die geringer ist als die Weite (W) der Mündung (25) des Schlitzes (24) (Figur 5).

Die Stege 21 sind wie Figur 5 zeigt, vorzugsweise von der Mündung 25 und somit von den Randkonturen 22, 23 beabstandet. Die Stege 21 gehen nahtlos in die Elektrodenplatte 19 über. Ebenso gehen die Stege 21 nahtlos in den Elektrodenträger 20 über. Die Länge der Stege 21 ist vorzugsweise zumindest so groß wie die Weite der Mündung 25.

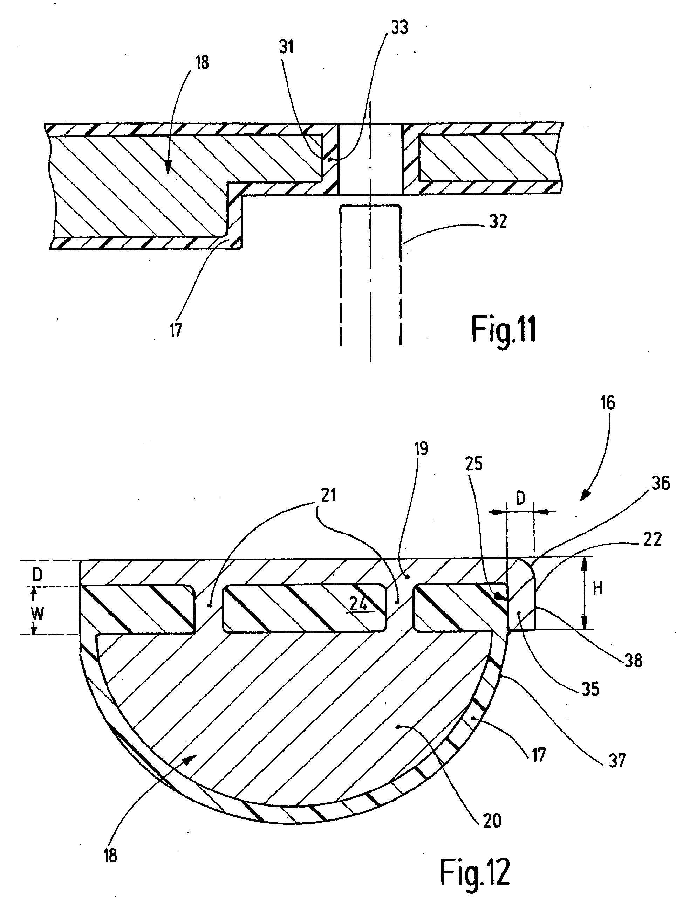

Die Randkontur 22 der Elektrodenplatte 19 kann, wie Figur 12 zeigt, eine Verlängerung in Form eines Vorsprungs 35 aufweisen. Dieser Vorsprung 35 ist in einem Winkel, vorzugsweise in einem 90° Winkel, zur Ebene der Elektrodenplatte 19 angeordnet und kann die Mündung 25 des Schlitzes 24 teilweise oder ganz überragen. Der Vorsprung 35 kann eine Höhe H aufweisen, die größer ist als die Dicke D der Elektrodenplatte. Die Höhe H des Vorsprungs 35 kann im Bereich von 0,3mm bis 0,5mm, vorzugsweise 0,4mm liegen. Die Dicke des Vorsprungs 35 ist vorzugsweise gleich der Dicke D der Elektrodenplatte 19, kann aber auch geringfügig größer oder kleiner sein. Der Vorsprung 35 überragt den Kunststoffmantel 17, sodass die äußere Kante 37 des Kunststoffmantels 17 in einem Abstand zur äußeren Kante 38 des Vorsprungs 35 angeordnet ist. Die äußere Kante 37 des Kunststoffmantels 17 ist im Vergleich zur äußeren Kante 38 des Vorsprungs 35 näher zur Mitte des Elektrodenträgers 20 angeordnet. Der Abstand zwischen der äußeren Kante 37 des Kunststoffmantels 17 und der äußeren Kante 38 des Vorsprungs 35 ist vorzugsweise etwas geringer als die Dicke D des Vorsprungs 35. Der Übergangsbereich 36 von Vorsprung 35 zur Elektrodenplatte 19 ist gewebeschonend vorzugsweise gerundet ausgebildet. Die Geometrie der Randkontur 22 mit dem Vorsprung 35 einer Branche 16 wie in Figur 12 an einer Stelle (auf der rechten Seite der Figur) dargestellt, erstreckt sich vorzugsweise entlang einer Flanke, dann über das distale Ende und dann entlang der gegenüber liegenden Flanke der Elektrodenplatte 19. Diese Randkontur 22 kann durchgehend kontinuierlich dieselbe Geometrie aufweisen, sie kann auch Unterbrechungen in Form von Ausnehmungen aufweisen. Eine Randkontur 22 mit Vorsprung 35 wie beschrieben unterstützt die sichere Verschließung von Gefäßen.

Der insoweit beschriebene Metallkörper, bestehend aus Elektrodenplatte 19, Elektrodenträger 20 und Stegen 21, ist vorzugsweise in einem additiven Fertigungsverfahren, beispielsweise pulvermetallurgisch durch Lasersintern bzw. Laserschmelzen (SLM-Verfahren), hergestellt. Damit haben die Stege 21 die gleiche Materialstruktur wie die Elektrodenplatte 19 und der Elektrodenträger 20 sowie die gleiche Festigkeit. Die Durchmesser der Stege 21 können geringer sein als die Länge der Stege 21. Ihre Querschnitte können zum Beispiel, wie in Figur 7 dargestellt, rund bzw. im Wesentlichen kreisförmig oder gemäß Figur 8 unrund ausgebildet sein. Auf diese Weise lassen sich hohe mechanische Festigkeit mit niedriger Wärmeleitfähigkeit paaren.

Vorzugsweise ist der Schlitz 24 mit einem Kunststoff ausgefüllt, der außen in den Kunststoffmantel 17 übergeht. Der Schlitz 24 ist somit verschlossen, so dass dem Eindringen von Flüssigkeit, Bakterien oder sonstigen biologischen Materialien entgegengewirkt wird. Der Kunststoff haftet außerdem an den einander zugewandten, den Schlitz 24 begrenzenden Flächen. Zusätzlich kann der Kunststoffmantel 17 an der rückseitigen in Figur 5 unteren Seite des Elektrodenträgers 20 gut haften. Der Kunststoffmantel 17 bewirkt eine elektrische und thermische Isolation des Elektrodenträgers 20 gegen umgebendes Gewebe. Der Kunststoff in dem Schlitz 24 bewirkt eine mechanische Unterstützung der Elektrodenplatte 19, sowie eine thermische Abschirmung derselben gegen den Elektrodenträger 20.

Figur 6 veranschaulicht eine Abwandlung des Querschnitts der Branche 16, insbesondere im Hinblick auf die Ausbildung der Stege 21 und des Elektrodenträgers 20. Dieser kann, wie dargestellt, etwa schalenförmig ausgebildet sein, wodurch die Länge der Stege 21 und die innere Weite des Schlitzes 24 größer werden. Die Stege 21 können zylinderartig oder auch an einem oder beiden Enden sich verdickend ausgebildet sein. Ansonsten gilt die obige Beschreibung unter Zugrundelegung gleicher Bezugszeichen entsprechend.

Anstelle filigraner Stege 21 können an einer oder mehreren Stellen auch Hohlstege 26 gemäß Figur 9 und 10 Anwendung finden. Diese Hohlstege 26 können einen Kanal 27 umschließen, der zum Beispiel die Oberseite der Elektrodenplatte 19 durchbricht und zur Aufnahme eines Abstandshalters 28, zum Beispiel aus Kunststoff, Keramik oder dergleichen, geeignet ist. Die Hohlstege 26 können gemäß Figur 10 eine Verbindung zwischen der Elektrodenplatte 19 und dem Elektrodenträger 20 bilden. Sie können jedoch auch als Blindzapfen ausgebildet sein, d.h. in einem Abstand vor dem Elektrodenträger 20 enden. In diesem Fall tragen sie nicht zur elektrischen und mechanischen Verbindung zwischen der Elektrodenplatte 19 und dem Elektrodenträger 20 bei. Die Verbindung wird dann ganz oder teilweise von anderen Stegen 21 und/oder dem indem Schlitz 24 angeordneten Kunststoff übernommen.

Der Kunststoffmantel 17 kann sich über den Elektrodenträger 20 hinaus bis zu einem Gelenkabschnitt 29 (Figur 3) und gegebenenfalls weiter zu einem Betätigungsanschluss 30 erstrecken. Der Gelenkabschnitt 29 dient zur Ausbildung eines Schwenkgelenks, um die Branchen 15, 16 nach Art einer Zange öffnen und schließen zu können. Dazu ist der Gelenkabschnitt 29 mit einer sich quer durch den Gelenkabschnitt 29 erstreckenden Durchgangsöffnung 31 versehen, die rund oder unrund ausgebildet sein kann und vorzugsweise einen Durchmesser aufweist, der größer ist als der Außendurchmesser eines zur Lagerung vorgesehenen Bolzens 32 (Figur 11). Der Kunststoffmantel 17 erstreckt sich vorzugsweise durch die Durchgangsöffnung 31 hindurch und bildet dort eine Lagerbuchse 33. Diese Lagerbuchse 33 ist einstückiger Bestandteil des Kunststoffmantels 17. Sie isoliert das Metallteil 18 gegen den Bolzen 32 und zentriert diesen zugleich in der Durchgangsöffnung 31 und zwar weitgehend unabhängig von Fertigungstoleranzen des Metallteils 18.

Das insoweit beschriebene Instrument 10 arbeitet wie folgt:

Die Elektrodenplatten 19 der beiden Branchen 15, 16 sind über durch den Schaft 11 führende Leitungen und ein Anschlusskabel 34 mit einer elektrischen Energiequelle, beispielsweise einem HF-Generator, verbunden. Bei Aktivierung ist zwischen den Elektrodenplatten 19 der Branchen 15, 16 eine elektrische Spannung vorhanden, so dass zwischen den Branchen 15, 16 gefasstes Gewebe bestromt wird. Dazu wird an dem Griff 14 ein Handhebel betätigt, um die Branchen 15, 16 zu schließen und Gewebe dazwischen zu fassen. Durch die Bestromung steigt die Temperatur im Gewebe, wodurch es koaguliert. Auch die Temperatur der Elektrodenplatten 19 steigt damit zum Teil über Siedetemperatur an. Die Wärme wird jedoch weitgehend auf die Elektrodenplatte 19 beschränkt. Der in dem Schlitz 24 angeordnete Kunststoff weist eine Wärmeleitfähigkeit auf, die geringer ist als die Wärmeleitfähigkeit der Elektrodenplatte 19. Zusätzlich übertragen die Stege 21 aufgrund ihrer geringen Querschnittsfläche nur wenig Wärmeenergie, so dass der Elektrodenträger 20 weitgehend kühl bleibt. Die bevorzugt große Wärmekapazität des Elektrodenträgers 20 nimmt die geringen übertragenen Wärmemengen mit nur geringer Temperaturerhöhung auf. Dieser Effekt kann verstärkt werden, indem in einer oder mehreren Hohlkammern des Kunststoffs und/oder des Elektrodenträgers 20 wärmepuffernde Materialien, insbesondere Latentwärmespeicher, zum Beispiel Wachs, angeordnet sind, wobei die Speichertemperatur vorzugsweise auf einen gewebeunschädlich niedrigen Temperaturbereich von zum Beispiel 60°C oder weniger festgelegt wird. Damit können die Außenseiten der Branchen 15, 16 auch bei längerem Einsatz hinreichend kühl gehalten werden.

Eine neuartige Branche 16 eines Instruments 10 weist ein einstückig nahtlos ausgebildetes Metallteil 18 auf, das sowohl den Elektrodenträger 20 wie auch die Elektrodenplatte 19 und vorhandene Verbindungsstege 21 umfasst. Vorzugsweise ist dieses Metallteil 18 in einem additiven Fertigungsverfahren, beispielsweise selektivem Laserschmelzen (SLM), hergestellt. Durch den Wegfall von Schweißpunkten oder Nähten zwischen der Elektrodenplatte 19 und dem Elektrodenträger 20 lassen sich mittels der Stege 21 schlecht wärmeleitende und gleichzeitig mechanisch sehr stabile Verbindungen schaffen. Die neuartige Branche eignet sich sowohl für offenchirurgische als auch für laparoskopische und flexibel endoskopische Instrumente.

### Bezugszeichenliste:

- 10: Instrument
- 11: Schaft
- 12: Werkzeug
- 13: Gehäuse
- 14: Griff
- 15: erste Branche
- 16: zweite Branche
- 17: Kunststoffmantel
- 18: Metallteil
- 19: Elektrodenplatte
- 20: Elektrodenträger
- 21: Stege
- 22: Randkontur der Elektrodenplatte
- 23: Randkontur des Elektrodenträgers
- 24: Schlitz
- 25: Mündung
- 26: Hohlsteg
- 27: Kanal
- 28: Abstandshalter
- 29: Gelenkabschnitt
- 30: Betätigungsanschluss
- 31: Durchgangsöffnung
- 32: Bolzen
- 33: Lagerbuchse
- 34: Anschlusskabel
- 35: Vorsprung
- 36: Übergangsbereich
- 37: äußere Kante von 17
- 38: äußere Kante von 35

## Patentansprüche

1. Elektrochirurgisches Instrument
mit einer Branche (16), die eine von einer Randkontur (22) begrenzte Elektrodenplatte (19) und einen ebenfalls von einer Randkontur (23) begrenzten Elektrodenträger (20) aufweist, die miteinander einen Schlitz (24) begrenzend angeordnet sind, wobei sich eine Mündung (25) des Schlitzes (24) zwischen den Randkonturen (22, 23) erstreckt, **dadurch gekennzeichnet, dass** die Elektrodenplatte (19) und der Elektrodenträger (20) über mehrere Stege (21) nahtlos einstückig miteinander verbunden sind.

2. Instrument nach Anspruch 1, **dadurch gekennzeichnet, dass** die Stege (21) von der Mündung (25) des Schlitzes (24) im Abstand angeordnet sind.

3. Instrument nach einem der vorigen Ansprüche, **dadurch gekennzeichnet, dass** der Abstand der Stege (21) von der Randkontur (23) des Elektrodenträgers (20) größer als die Weite (W) der Mündung (25) des Schlitzes (24) ist.

4. Instrument nach einem der vorigen Ansprüche, **dadurch gekennzeichnet, dass** die Stege (21) einen Rundquerschnitt aufweisen.

5. Instrument nach einem der vorigen Ansprüche, **dadurch gekennzeichnet, dass** die Stege (21) einen größten Durchmesser aufweisen, der kleiner ist, als die Weite (W) der Mündung(25) des Schlitzes.

6. Instrument nach einem der vorigen Ansprüche, **dadurch gekennzeichnet, dass** der Elektrodenträger (20) einen schalenförmigen Querschnitt aufweist.

7. Instrument nach einem der vorigen Ansprüche, **dadurch gekennzeichnet, dass** die Elektrodenplatte (19) an einem Ende nahtlos in den Elektrodenträger (20) übergeht.

8. Instrument nach einem der vorigen Ansprüche, **dadurch gekennzeichnet, dass** die Elektrodenplatte (19) als ebenes Teil ausgebildet ist.

9. Instrument nach einem der vorigen Ansprüche, **dadurch gekennzeichnet, dass** die Elektrodenplatte (19) eine Profilierung aufweist.

10. Instrument nach einem der vorigen Ansprüche, **dadurch gekennzeichnet, dass** die Elektrodenplatte (19) eine Dicke (D) aufweist, die geringer ist als die Weite (W) der Mündung (25) des Schlitzes (24).

11. Instrument nach einem der vorigen Ansprüche, **dadurch gekennzeichnet, dass** der Elektrodenträger (20), die Stege (21) und die Elektrodenplatte (19) in einem additiven oder generativen Fertigungsverfahren aus einem einheitlichen Material bereitgestellt sind.

12. Instrument nach einem der vorigen Ansprüche, **dadurch gekennzeichnet, dass** der Schlitz (24) mit einem Material gefüllt ist dessen Wärmeleitfähigkeit geringer ist als die Wärmeleitfähigkeit des Materials der Elektrodenplatte (19).

13. Instrument nach einem der vorigen Ansprüche, **dadurch gekennzeichnet, dass** der Elektrodenträger (20) mit einem elektrisch isolierenden Material umhüllt ist.

14. Instrument nach einem der vorigen Ansprüche, **dadurch gekennzeichnet, dass** der Elektrodenträger (20) eine Durchgangsöffnung (31) zur Ausbildung einer Lagerbohrung aufweist.

15. Instrument nach Anspruch 14, **dadurch gekennzeichnet, dass** die Durchgangsöffnung (31) mit einem elektrisch isolierenden Material ausgekleidet ist.

## Claims

1. Electrosurgical instrument, comprising a branch (16) which has an electrode plate (19) delimited by an edge contour (22) and an electrode carrier (20) delimited by an edge contour (23), which are arranged with one another in such a way as to delimit a slot (24), wherein a mouth (25) of the slot (24) extends between the edge contours (22, 23), **characterized in that** the electrode plate (19) and the electrode carrier (20) are connected to one another seamlessly in one piece via a plurality of webs (21).

2. Instrument according to claim 1, **characterized in that** the webs (21) are arranged at a distance from the mouth (25) of the slot (24).

3. Instrument according to any of the preceding claims, **characterized in that** the distance of the webs (21) from the edge contour (23) of the electrode carrier (20) is greater than the width (W) of the mouth (25) of the slot (24) .

4. Instrument according to any of the preceding claims, **characterized in that** the webs (21) have a round cross-section.

5. Instrument according to any of the preceding claims, **characterized in that** the webs (21) have a largest diameter which is smaller than the width (W) of the mouth (25) of the slot.

6. Instrument according to any of the preceding claims, **characterized in that** the electrode carrier (20) has a bowl-shaped cross-section.

7. Instrument according to any of the preceding claims, **characterized in that** the electrode plate (19) merges seamlessly at one end into the electrode carrier (20).

8. Instrument according to any of the preceding claims, **characterized in that** the electrode plate (19) is configured as a flat part.

9. Instrument according to any of the preceding claims, **characterized in that** the electrode plate (19) has a profiling.

10. Instrument according to any of the preceding claims, **characterized in that** the electrode plate (19) has a thickness (D) which is smaller than the width (W) of the mouth (25) of the slot (24).

11. Instrument according to any of the preceding claims, **characterized in that** the electrode carrier (20), the webs (21) and the electrode plate (19) are provided in an additive or generative production method from a single material.

12. Instrument according to any of the preceding claims, **characterized in that** the slot (24) is filled with a material having a thermal conductivity which is lower than the thermal conductivity of the material of the electrode plate (19).

13. Instrument according to any of the preceding claims, **characterized in that** the electrode carrier (20) is covered with an electrically insulating material.

14. Instrument according to any of the preceding claims, **characterized in that** the electrode carrier (20) has a through-opening (31) for forming a bearing bore.

15. Instrument according to claim 14, **characterized in that** the through-opening (31) is lined with an electrically insulating material.

## Revendications

1. Instrument électrochirurgical doté d'un mors (16) qui présente une plaque d'électrode (19), délimitée par un contour de bord (22), et un support d'électrode (20) également délimité par un contour de bord (23), qui sont disposés de façon à délimiter ensemble une fente (24), une ouverture (25) de la fente (24) s'étendant entre les contours de bord (22, 23), **caractérisé en ce que** la plaque d'électrode (19) et le support d'électrode (20) sont reliés l'un à l'autre d'une seule pièce, sans soudure, par plusieurs entretoises (21).

2. Instrument selon la revendication 1, **caractérisé en ce que** les entretoises (21) sont disposées à distance de l'ouverture (25) de la fente (24).

3. Instrument selon l'une des revendications précédentes, **caractérisé en ce que** la distance des entretoises (21) par rapport au contour de bord (23) du support d'électrode (20) est supérieure à la largeur (W) de l'ouverture (25) de la fente (24).

4. Instrument selon l'une des revendications précédentes, **caractérisé en ce que** les entretoises (21) présentent une section ronde.

5. Instrument selon l'une des revendications précédentes, **caractérisé en ce que** les entretoises (21) présentent un diamètre maximal qui est inférieur à la largeur (W) de l'ouverture (25) de la fente.

6. Instrument selon l'une des revendications précédentes, **caractérisé en ce que** le support d'électrode (20) présente une section transversale en forme de cuvette.

7. Instrument selon l'une des revendications précédentes, **caractérisé en ce que** la plaque d'électrode (19) se raccorde par une extrémité sans soudure au support d'électrode (20).

8. Instrument selon l'une des revendications précédentes, **caractérisé en ce que** la plaque d'électrode (19) est réalisée comme élément plan.

9. Instrument selon l'une des revendications précédentes, **caractérisé en ce que** la plaque d'électrode (19) présente un profil.

10. Instrument selon l'une des revendications précédentes, **caractérisé en ce que** la plaque d'électrode (19) présente une épaisseur (D) qui est plus faible que la largeur (W) de l'ouverture (25) de la fente (24).

11. Instrument selon l'une des revendications précédentes, **caractérisé en ce que** le support d'électrode (20), les entretoises (21) et la plaque d'électrode (19) sont préparés à partir d'un matériau homogène lors d'un procédé de fabrication additif ou génératif.

12. Instrument selon l'une des revendications précédentes, **caractérisé en ce que** la fente (24) est remplie avec un matériau dont la conductivité thermique est plus faible que la conductivité thermique du matériau de la plaque d'électrode (19).

13. Instrument selon l'une des revendications précédentes, **caractérisé en ce que** le support d'électrode (20) est enveloppé d'un matériau électriquement isolant.

14. Instrument selon l'une des revendications précédentes, **caractérisé en ce que** le support d'électrode (20) présente une ouverture de passage (31) destinée à former un alésage de palier.

15. Instrument selon la revendication 14, **caractérisé en ce que** l'ouverture de passage (31) est garnie d'un matériau électriquement isolant.
